# EUROPEAN PATENT APPLICATION

(11) **EP 3 913 049 A1**
(43) Date of publication of application: **24.11.2021**
(21) Application number: 20176196.2
(22) Date of filing: 22.05.2020
(51) Int. Cl.: C12N 5/0783, C07K 14/72, C12N 15/113

(54) **VIRUS-SPECIFIC GLUCOCORTICOID-RESISTANT T CELLS FOR ADOPTIVE T-CELL THERAPY AND METHODS FOR GENERATING THEREOF**

(71) Applicant: Klinikum der Universität München Anstalt des öffentlichen Rechts, 81377 München (DE)
(72) Inventor: FEUCHTINGER, Tobias, 81929 München (DE); KÄUFERLE, Theresa, 80801 München (DE)
(74) Representative: Vossius & Partner Patentanwälte Rechtsanwälte mbB

(57) **Abstract**

The present invention relates to a method for generating virus-specific glucocorticoid-resistant T cells, comprising: (a) enriching virus-specific T cells *in vitro* from peripheral blood mononuclear cells (PBMCs), wherein a virus-specific T cell is characterized by an elevated expression and/or secretion level of one or more T cell-specific activation markers and/or pro-inflammatory cytokines in response to a prior stimulation with one or more virus-specific antigens as compared to without stimulation with viral-specific antigens; and (b) disrupting the functionality of the endogenous glucocorticoid receptor (GR) in the enriched virus-specific T cells by introducing one or more nucleotide base insertions and/or deletions ('InDels') into the GR encoding gene.

## Description

The present invention relates to a method for generating virus-specific glucocorticoid-resistant T cells, comprising: (a) enriching virus-specific T cells *in vitro* from peripheral blood mononuclear cells (PBMCs), wherein a virus-specific T cell is characterized by an elevated expression and/or secretion level of one or more T cell-specific activation markers and/or pro-inflammatory cytokines in response to a prior stimulation with one or more virus-specific antigens as compared to without stimulation with viral-specific antigens; and (b) disrupting the functionality of the endogenous glucocorticoid receptor (GR) in the enriched virus-specific T cells by introducing one or more nucleotide base insertions and/or deletions ('InDels') into the GR encoding gene.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Allogeneic hematopoietic stem cell transplantation (HSCT) and solid organ transplantation (SOT) are curative treatments for a variety of diseases (such as leukemia, multiple myeloma, immune deficiency syndromes or myelodysplastic syndrome), but expose patients to periods of marked immunosuppression. Refractory viral infections cause life-threatening conditions due to the deficient T-cell response post transplantation, a situation for which effective treatment options are urgently needed.^{1, 2, 3, 4} In allogeneic HSCT for example, reconstitution of T-cell immunity from the stem cell donor can take up to several months, so that refractory viremia related mortality in these patients is up to 50%. Whereas in healthy individuals, persistent viruses like Cytomegalovirus (CMV) are often asymptomatic or cause only mild symptoms and persist lifelong in the human body, CMV infection or re-activation in the immunocompromised host can lead to symptomatic disease which most commonly includes interstitial pneumonitis, hepatitis or gastrointestinal symptoms (Schuster et al. (2005), Klin Padiatr 217 Suppl. 1: S67-84). Highest mortality rates have been described among recipients of T-cell depleted graft or transplant from unrelated or HLA-mismatched donors refractory to standard antiviral therapy (Boeckh and Nichols 2004 Blood 103(6):2003-2008; Kapp et al., (2007) Cytotherapy 9(8): 699-711; Koehl et al., (2008), Klin Padiatr 220(6): 348-352). Protective T-cell immunity can be restored by means of adoptive T-cell transfer, generating virus-specific T-cell products from healthy donor cells.⁵ Over the past years it has been demonstrated that this approach can be applied for patients with refractory viral infections after stem cell transplantation.^{6, 7, 8, 9} In evidence-based treatment guidelines, T-cell therapy has become a second line treatment recommendation.^{10, 11}

Virus-specific T-cell products can be generated by *in vitro* stimulation and expansion ^{12, 13} or selection techniques. Selection has become possible by efforts made to directly isolate virus-specific T cells from peripheral blood of seropositive donors in order to induce efficient proliferation under physiological conditions *in vivo.* For direct selection of virus-specific T cells, cytokine capture technique could be applied, leading to a combination of virus-specific Tₕₑₗₚₑᵣ as well as cytotoxic T cells.⁶ Isolation based on streptamer technology leads to high purity of peptide-specific CD8⁺ virus-specific T-cell products.¹⁴ Directly isolated virus-specific T cells are obtained in small amounts and are infused into the patient where they can expand effectively upon the presence of viral antigen *in vivo.* This physiological re-stimulation and T-cell response induce viral clearance as well as sustained protection.

However, this treatment undergoes immediate inactivation *in vivo* in those patients receiving relevant immunosuppression like patients with graft versus host disease (GvHD) or patients after SOT. In many cases the immunosuppression itself is the major cause of the uncontrolled viral infection. Patients with significant graft-versus-host disease (GvHD) *post* stem cell transplantation are at highest risk of recurrent and refractory viral infections, largely because of high-dose glucocorticoid therapy.¹⁵ Since glucocorticoids exert potent immunosuppressive effects, they remain the most effective and favored initial therapy for the treatment of acute and chronic GvHD. However, glucocorticoid treatment provides a major obstacle to adoptive T-cell therapy, since glucocorticoids upon engagement with the endogenous glucocorticoid receptor (GR) trigger signaling cascades which severely suppress activation and expansion¹⁶ of transferred T cells, and consequently inhibit T cell-mediated effector functions which would be required for combating viral infection.¹⁷

Thus, there is an urgent need in the art for more effective means and methods for successfully treating and/or preventing viral infection and/or associated disorders in patients receiving ongoing glucocorticoid-mediated immunosuppressive therapy. The present invention addresses this need and provides means and methods useful for such treatments.

Accordingly, the present invention relates in a first aspect to a method for generating virus-specific glucocorticoid-resistant T cells, comprising: (a) enriching virus-specific T cells *in vitro* from peripheral blood mononuclear cells (PBMCs), wherein a virus-specific T cell is characterized by an elevated expression and/or secretion level of one or more T cell-specific activation markers and/or pro-inflammatory cytokines in response to a prior stimulation with one or more virus-specific antigens as compared to without stimulation with viral-specific antigens; and (b) disrupting the functionality of the endogenous glucocorticoid receptor (GR) in the enriched virus-specific T cells by introducing one or more nucleotide base insertions and/or deletions ('InDels') into the GR encoding gene.

The term "virus-specific antigen", as used herein, refers to a molecule of any molecular class (including nucleic acids, proteins, lipids, glycans, protein-glycan-conjugates and glycan-lipid-conjugates) that is specifically found in a certain virus, type of virus, or group of viruses; and which bears one or more epitopes which can be recognized by a T cell (i.e., a T cell receptor expressed on said T-cell). Preferably, a virus-specific antigen is a virus protein or protein-glycan conjugate, more preferably a virus surface protein or fragment thereof, or a virus surface glycoprotein or fragment thereof; and wherein the fragment is preferably a peptide of 8 to 15 amino acids length (optionally carrying one or more glycans as modification).

In a preferred embodiment of the invention, the one or more virus-specific antigens are from one or more different viruses (or type or subtypes of viruses).

The term "nucleic acid", as interchangeably used herein with the term "nucleic acid molecule", refers to polynucleotides including deoxyribonucleic acid (DNA), such as cDNA or genomic DNA, and, where appropriate, ribonucleic acid (RNA). It is understood that the term "RNA" as used herein comprises all forms of RNA including mRNA.

The term "protein" in accordance with the present invention describes a group of molecules which comprises the group of peptides, consisting of up to 30 amino acids, as well as the group of polypeptides, consisting of more than 30 amino acids. Proteins may further form dimers, trimers and higher oligomers, i.e., consisting of more than one protein molecule. Protein molecules forming such dimers, trimers etc. may be identical or non-identical. The corresponding higher order structures are, consequently, termed homo- or heterodimers, homo- or heterotrimers etc. Homo- or heterodimers etc. also fall under the definition of the term "protein". The term "protein" also refers to chemically or posttranslationally modified peptides and polypeptides.

"T cells" or "T lymphocytes", as interchangeably used herein, are a subset of lymphocytes (a subtype of white blood cell) that originate from haematopoietic stem cells produced in the bone marrow and migrate to the thymus for maturation and play a central role in cell-mediated immunity. T cells can be distinguished from other lymphocytes, such as B cells and natural killer (NK) cells, e.g., by the presence of a T-cell receptor (TCR) on their cell surface. T cells include, for example, natural killer T (NKT) cells, cytotoxic T lymphocytes, T helper cells, and regulatory T (Treg) cells.

The term "virus-specific T cell", as used herein, refers to a T cell which expresses and bears on its surface one or more TCRs that is capable of specifically recognizing one or more virus-specific antigens.

T cell responses can be detected by analysis for their effects on other cells (e.g., elimination of target cells, macrophage activation, activation of B cells), their proliferation capacity upon specific stimulation, or by the cytokines they produce.

The term "glucocorticoid-resistant T cell", as used herein, refers to a T cell, which, on account of its disrupted GR functionality (as described below), is substantially resistant to the immunosuppressive effects of glucocorticoids.

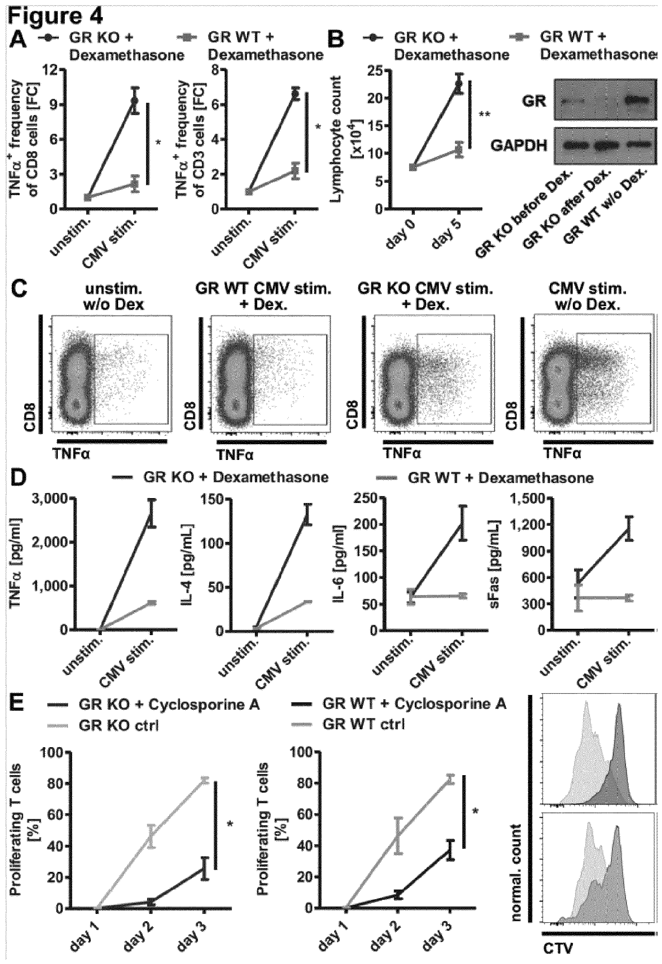

For instance, whereas in T cells with functional GR expression glucocorticoids have an inhibitory effect on the production/secretion of pro-inflammatory cytokines, in "glucocorticoid-resistant T cells" this production/secretion is substantially maintained in the presence of glucocorticoids; see Fig. 4A. Moreover, whereas glucocorticoids typically have an anti-proliferative effect on T cells with functional GR expression, the proliferation/expansion capacity of a "glucocorticoid-resistant T cell" is substantially maintained in the presence of glucocorticoids; see Fig. 4B.

The term "peripheral blood mononuclear cell" or "PBMC", as used herein, refers to a population of white blood cells having a round nucleus, i.e., including lymphocytes (T cells, B cells and NK cells) and monocytes, which has not been enriched for a given sub-population. PBMCs may be obtained from whole blood samples by any suitable method known in the art. By way of example, PBMCs can be extracted from whole blood using Ficoll, a hydrophilic polysaccharide that separates layers of blood, with the PBMC forming a cell ring under a layer of plasma. Additionally, PBMC can be extracted from whole blood using a hypotonic lysis buffer, which will preferentially lyse red blood cells. For the clinical setting, white blood cells can also be isolated from a donor's whole blood by leukapheresis.

The term "T cell-specific activation marker and/or pro-inflammatory cytokine" as used herein refers to any gene product, including mRNA and/or protein, of a T cell, whose expression and/or secretion is elevated (i.e., upregulated) in response to a prior stimulation with one or more viral antigens as compared without stimulation. The skilled person understands that particularly such T cell gene products are suitable for the method of the invention which are characteristically (ideally predominantly, not necessarily exclusively) expressed by antigen-activated T cells (particularly on CD8⁺ and/or CD4⁺ T cells), and that are not expressed by other cell types present in the PBMC sample. Examples of T cell-specific activation markers include, but are not limited to, CD25, CD69 and CD95 (Fas). Examples of T cell-specific pro-inflammatory cytokines include, but are not limited to, Interferon gamma (IFNγ), tumor necrosis factor alpha (TNF-α), tumor necrosis factor alpha (TNF-β), and Interleukin-2, -3, -6, and -8.

The expression and/or secretion of activation markers and/or pro-inflammatory cytokines can be measured by methods known to those skilled in the art, including, for example, Western blot analysis, Northern blot analysis, RT-PCR, ELISA, bead-based multiplexarrays, immunofluorescence analysis, and flow-cytometry-based cell classification analysis.

The term "prior stimulation with one or more virus-specific antigens", as used herein, means that T cells comprised in the PBMCs have been in prior contact with one or more virus-specific antigens, i.e., either (i) *in vivo*, *i*.*e*., within the body of the donor from which the PBMCs originate, wherein the donor is, or was, carrying an infection with one or more corresponding viruses (from which the one or more virus-specific antigens derive); and/or wherein the donor previously received virus-specific antigen, e.g. in the context of a vaccination/immunization; or (ii) *in vitro, i.e.,* after the blood sample comprising the PBMCs has been obtained from the donor's body. It is understood that said "prior contact" must have occurred at a sufficient dose and for a sufficient amount of time to trigger activation of those T cells specifically recognizing the one or more virus-specific antigens; and that the time period between said prior contact and the enriching in step (a) is short enough so that at least a substantial amount of said T cells is still in the required activated state, i.e., characterized by an elevated expression and/or secretion of one or more T cell-specific activation markers and/or pro-inflammatory cytokines.

Thus, in a preferred embodiment of the first aspect of the present invention, the prior stimulation with one or more virus-specific antigens is conducted *in vitro* and for a duration of between about, with increasing preference, 0.25 hours to 48 hours, 0.5 hours to 24 hours, 1 hour to 20 hours, 2 hours to 16 hours, 2.25 hours to 12 hours, 2.5 hours to 10 hours, 2.75 hours to 8 hours, 3 hours to 6 hours, or 3.25 hours to 5 hours, or 3.5 to 4 hours, prior to the enriching. In the appended Examples, a stimulation of the PBMCs (with CMV pp65-derived antigens) proved particularly effective when conducted for 4 hours. However, other (i.e., longer and/or shorter) time periods may also be suitable in particular cases and deviations from the above specified time periods are also envisaged.

The term "elevated expression and/or secretion level", as used herein, refers to any detectable increase of an expression and/or secretion level as compared to a reference (e.g., a T cell which has not been exposed to a prior stimulation with a virus-specific antigen).

Preferably, an "elevated expression and/or secretion level" of one or more T cell-specific activation markers and/or pro-inflammatory cytokines "as compared to without stimulation" means that the detected expression or secretion level of said one or more T cell-specific activation markers and/or pro-inflammatory cytokines is, with increasing preference, at least 10%, at least 20%, at least 30%, at least 40%, at least 50%, at least 60%, at least 70%, at least 80%, at least 85%, at least 90%, or at least 95% higher as compared to a reference sample comprising a comparable amount of cells of similar type or types which have not been stimulated with the respective antigen.

The expression and/or secretion of activation markers and/or pro-inflammatory cytokines can be measured by methods known to those skilled in the art, including, for example, Western blot analysis, Northern blot analysis, RT-PCR, Elispot, bead-based multiplexarrays, immunofluorescence analysis, and flow-cytometry-based cell classification analysis.

The term "enriching" or "enrichment", as interchangeably used herein, refers generally to any isolation or sorting process that increases the relative abundance of a desired cell type, or cell types, in a population of cells. Enrichment may be achieved by any method known in the art. Exemplary enrichment methods are, without being limiting, cell sorting approaches such as flow cytometry activated cell sorting (FACS) or magnetic activated cell sorting (MACS) including microfluidic-based approaches, panning approaches using immobilized antibodies, high-throughput fluorescence microscopy, or density gradient approaches. All these methods are known to the person skilled in the art and have been described, for example, in the Review by Dainiak MB, et al., Adv Biochem Eng Biotechnol. (2007);106:1-18.

Others have recently, in parallel with the present inventors, endorsed the concept of generating virus-specific glucocorticoid-resistant T cells by genetic knockout of the glucocorticoid receptor (GR) using transcription activator-like effector nuclease (TALEN); see Ref.²¹. However, in this previous study, the genetic knockout has been conducted in Streptamer-selected T cells. This approach suffers from severe disadvantages: firstly the selection is HLA-restricted, secondly only available for known immunodominant epitopes and thirdly generated T-cell products are restricted to CD8⁺ T cells only. Consequently, this approach has not reached clinical routine application to date.

Here, the present inventors set out to develop a method for generating virus-specific glucocorticoid-resistant T-cells suitable for adoptive T cell therapy that overcomes these previous impediments by combining a particular selection approach which specifically selects activated T cells (i.e., based on their activated status, as manifested by an elevated expression and/or secretion of one or more activation markers and/or pro-inflammatory cytokines) with a gene-knockout of the endogenous GR (GR-KO) by using a nuclease-based gene editing technology such as CRISPR/Cas9.

This combinatorial cell selection and engineering approach overcomes the hurdles of previous approaches including HLA-restriction, limited immunodominant epitopes and is compatible with requirements for GMP grade engineering in clinical studies. Inducing GR-KO T cell responses opens a counterintuitive clinical scenario for the treatment and/or prevention of recurrent or refractory viral infection in patients receiving immunosuppressive therapy, wherein immunosuppression can be switched transiently from any other drug to glucocorticoids (such as dexamethasone) until recovery from the viral infection.

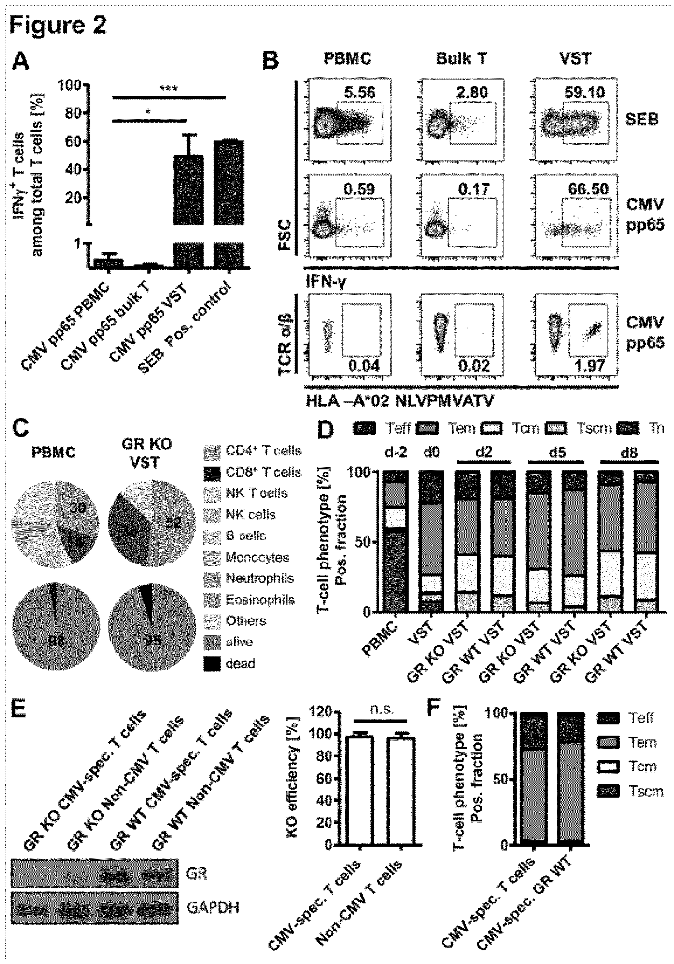

Moreover, the inventors found that the virus-specific glucocorticoid-resistant T cells generated by applying the method of the invention surprisingly possess superior viability (i.e., ratio of alive cells vs. dead cells) as compared to the virus-specific glucocorticoid-resistant T cell products obtained by previous approaches (e.g. Menger *et al.*²¹). In particular, in appended Example 2 and corresponding Figure 2C, it is shown that the virus-specific glucocorticoid-resistant T cells obtained by the method of the invention possessed a mean viability of 95% (n=4 donors). Viability was determined after step (b) by using a no-lyse-no-wash protocol (excluding any centrifugation or washing steps, which could change the cellular composition) via 7-AAD staining and subsequent flow-cytometric analysis. Viability is depicted as 7-AAD negative cells among leucocytes. Without wishing to be bound by any theory, it is believed by the present inventors that this superior viability is on account of synergistic effects resulting from the combination of the activation-based selection and GR gene-knockout as employed by the method of the invention.

Thus, in accordance with a preferred embodiment of the first aspect of the invention, the virus-specific glucocorticoid-resistant T cells obtained by the method of the invention have a viability (ratio of alive cells vs. total cells) of, with increasing preference, at least 65%, at least 70%, at least 75%, at least 80%, at least 85%, at least 90%, or at least 95% or higher viability of the total amount of cells obtained by the method of the invention, wherein preferably the viability is assessed at a time point within, with increasing preference, about 1 hour to 10 days, 5 hours to 8 days, 12 hours to 6 days, 1 day to 4 days, 1.5 days to 2 days, or most preferably at a time point of about 2 days after disrupting the functionality of the GR (i.e., by conducting the GR knockout) in step (b). It is understood that, in cases where step (b) implements an electroporation step (e.g., for introducing a CRISPR/Cas9 system into the enriched virus-specific T cells), the viability is determined at a time point within any of these defined intervals after conducting the electroporation.

Methods for assessing the viability of T cells are known in the art and described in the appended Examples.

In accordance with another preferred embodiment of the first aspect of the invention, the enriching in step (a) selectively enriches virus-specific T cells based on their activated cellular status, wherein the activated cellular status is manifested by an/the elevated expression and/or secretion level of one or more T cell-specific activation markers and/or pro-inflammatory cytokines in response to a prior stimulation with one or more virus-specific antigens as compared to without stimulation with viral-specific antigen(s).

In accordance with a further preferred embodiment of the first aspect of the invention, the enriching in step (a) comprises (i) applying a first binding agent which specifically binds to the one or more T cell-specific activation markers and/or pro-inflammatory cytokines, wherein said one or more activation markers and/or cytokines are immobilized on the T cell surface, (i-1) because of being naturally associated (e.g. membrane-anchored) with the T cell surface, or (i-2) because of being bound to the T cell surface via the first binding agent, which (bi-specifically) binds to a T cell-specific cell surface marker and to the one or more activation markers and/or cytokines; and (ii) enriching the virus-specific T cells by (ii-1) capturing the first binding agent; or (ii-2) applying a second binding agent which specifically binds to the one or more activation markers and/or cytokines that are immobilized on the T cell surface by the first binding agent, and capturing the second binding agent; and wherein said capturing of the first binding agent or of the second binding agent is established with a capturing agent or matrix interacting with a capturing moiety that is comprised (intrinsic or conjugated) in the first binding agent or the second binding agent.

The term "binding agent" as used herein refers to a molecular structure that binds specifically to an antigen or to another molecular structure (e.g., protein, lipid, DNA, RNA, carbohydrate, and/or a part or modification thereof). Non-limiting examples of a binding agent include antibodies (e.g., antibodies immunologically or genetically derived from any species (e.g., human, chicken, camel, llama, lamprey, shark, goat, rodent, cow, dog, rabbit, etc.), antibody domains or parts thereof (e.g., Fab, Fab', F(ab')₂, Fv, scFv, VH, VHH, VL, VLRs, the like), diabodies, monoclonal antibodies, serum, polyclonal Abs, mAbdAbs, phage display derived binders, affibodies, heteroconjugate antibodies, bispecific antibodies, evibodies, lipocalins, affibodies, avimers, maxibodies, heat shock proteins such as GroEL and GroES, trans-bodies, DARPins, aptamers, C-type lectin domains (e.g., tetranectins); human γ-crystallin and human ubiquitin-derived binders (e.g., affilins), PDZ domain-derived binders; scorpion toxin and/or kunitz type domain binders, fibronectin-derived binders (e.g., adnectins), receptors, ligands, lectins, streptavidin, biotin, derivatives or combinations thereof.

In accordance with a more preferred embodiment of the first aspect of the invention, the enriching in step (a) is not conducted based on a binding interaction between one or more TCRs expressed on said T cells and virus-specific antigen.

Preferably, cells are enriched such that after the enriching at least 10% of the cell population are virus-specific T cells. More preferable, at least 15%, at least 20%, at least 25%, such as at least 30%, at least 35%, at least 40%, at least 45%, at least 48%, such as at least 49% and most preferably 50% of the cell population are virus-specific T cells.

It is noted that the approach described by Menger *et al*.²¹ employed a streptamer-based selection which is restricted to CD8+ T cells; and irrespective of the cellular activation status of the T cells. In contrast, the selection employed by the present invention selects only activated T cells by targeting one or more activation markers and/or pro-inflammatory cytokines expressed at elevated level from these activated T cells and thereby enriches an activated T cell population which at least comprises CD8⁺ cytotoxic T cells and CD4⁺ helper T cells. The additional presence of the latter T cell subtype and the isolation of activated cells only may further contribute to the observed enhanced viability (as discussed above).

In accordance with the first aspect of the invention, the virus-specific T cells enriched in step (a), comprise CD8⁺ cytotoxic T-cells and CD4⁺ helper T-cells.

In other words, in accordance with the first aspect of the invention, the enriching in step (a) enriches CD8⁺ cytotoxic T-cells and CD4⁺ helper T-cells.

The term "CD4" as referred to herein is a glycoprotein expressed on the surface of T helper cells, regulatory T cells, monocytes, macrophages, and dendritic cells. CD4 was originally known as leu-3 and T4 (after the OKT4 monoclonal antibody).

A "CD4+ T cell" or "CD4 T lymphocyte" as referred to herein is a lymphocyte that expresses the CD4 glycoprotein on its surface. CD4+ T cells include helper T cells (CD4+ helper T cells), which are T cells that help orchestrate the immune response, including antibody responses and killer T cell responses. CD4+ T cell precursors differentiate into one of several subtypes, including TH1 (type 1 helper T cell), TH2 (type 2 helper T cell), TH3 (T helper 3 cells), TH17 (T helper 17 cells) or TFH (Follicular B helper T cells). These subtypes of helper T cells are characterized by their secretion of different cytokines to facilitate different types of immune responses. In certain embodiments, a CD4+ T cell is an effector T cell.

An "effector T cell" as referred to herein is a T cell that has been activated by its cognate antigen (i.e., here a virus antigen), and is actively involved in eliminating a pathogen (e.g. one or more viruses). Thus, an effector T cell actively responds to a stimulus (a pathogen or a co-stimulation) and carries out a cell-mediated immune response. Non-limiting examples of effector T cells as referred to herein include helper T cells, cytotoxic T cells (killer T cells) and regulatory T cells.

The term "CD8", as referred to herein, relates to a transmembrane glycoprotein that acts as a co-receptor for the T cell receptor (TCR). Like the TCR, CD8 binds to a major histocompatibility complex (MHC) molecule, but is specific for the class I MHC protein.

A "CD8+ T lymphocyte" or "CD8 T cell" as referred to herein is a lymphocyte that expresses the CD8 glycoprotein on its surface. Examples of CD8+ T cells include cytotoxic T cells (also referred to as "CD8⁺ cytotoxic T cells) and natural killer cells.

The term "glucocorticoid receptor" ("GR"), also known in the art as GCR or NR3C1 (nuclear receptor subfamily 3, group C, member 1), refers to a family of intracellular receptors to which cortisol and other glucocorticoids (alternatively called glucocorticosteroids, corticosteroids, or steroids) bind. GR acts as a ligand-activated transcription factor, which upon ligand binding, translocates to the nucleus where it exerts its immunosuppressive functions by having direct and indirect effects on gene expression. It is understood that the term "GR" as used herein also includes all known isoforms (including splice variants) and homologs.

The term "disrupting the functionality" of the endogenous GR means that one or more mutations (i.e., nucleotide base insertions and/or deletions ('InDels')) are introduced into the GR encoding gene (e.g., by means of targeted gene editing) by which the reading frame is disrupted, thereby preventing the functional expression of the GR protein.

Methods for introducing such disrupting mutations into a target gene (i.e., the GR encoding gene) in living mammalian cells (such as T cells) are known in the art and described below. Respective methods particularly envisaged by the present invention, without being limiting, are nuclease-based gene editing methods including, with increasing preference, zinc finger nucleases (ZFNs), transcription activator-like effector nucleases (TALENs), and Clustered Regulatory Interspaced Short Palindromic Repeat (CRISPR)/Cas-based RNA-guided DNA endonucleases, as discussed, e.g., in Gaj T. et al. (2013); Trends Biotechnol. 31(7): 397-405; Carroll D. (2012); Molecular therapy 20(9):1659-1660; Xiao A. et al. (2013), Nucleic Acids Research.1-11. The application of these three nuclease-based gene-editing methods in the particular context of disrupting a target gene in T cells has been previously reported, for example, in Ref¹⁸.

In accordance with a preferred embodiment of the invention, the GR encoding gene is the Homo sapiens NR3C1 gene defined by NCBI Reference Sequence: NG_009062.1.

In accordance with a preferred embodiment of the first aspect of the invention, the enriching in (a) comprises: (i) applying a first binding agent which specifically binds to a T cell-specific surface-molecule and to the one or more activation markers or cytokines; (ii) applying a second binding agent, which (ii-1) specifically binds to the activation marker and/or cytokine that is/are bound by the first binding agent; and (ii-2) comprises a capturing moiety which is capable of reversibly interacting with a capturing agent or matrix; and/or comprises a fluorescent and/or magnetic label for cell sorting; and (iii) enriching the virus-specific T cells by applying (iii-1) a capturing method, wherein the virus-specific T cells are captured based on the interaction between the capturing moiety and the capturing agent or matrix; and wherein uncaptured cells are removed; and/or (iii-2) a cell sorting, wherein the cells are sorted based on the presence of a fluorescent and/or magnetic label.

The term "T cell-specific cell surface molecule", "T cell-specific marker" or "T-cell marker" as interchangeably used herein, refers to a cell surface molecule (preferably a protein) which is characteristically (ideally selectively) expressed on T cells (or a particular T cell subtype or maturation state). Preferably, said surface molecule is a membrane-anchored or transmembrane receptor. T cell markers suitable for use in the present invention include, but are not limited to CD45, CD3, CD4, CD8, or any other surface molecule specific for T cells.

In a preferred embodiment of the first aspect of the invention, the T cell-specific cell surface molecule is CD45.

The term "CD45", also known as "protein tyrosine phosphatase receptor type C (PTPRC)" or "leukocyte common antigen (LCA)", refers to a protein tyrosine phosphatase (PTP) acting as an essential regulator of T-cell antigen receptor signaling.

The term "capturing moiety", as used herein, refers to a molecular portion or entity which can reversibly interact with a particular "capturing agent or matrix". Thus, a "capturing moiety" and a "capturing agent or matrix" may refer to respective members of a binding pair whose interaction can be used to perform a capturing of a desired cell type or population and for separation (depletion, or removal) of undesired cells. A capturing moiety may be intrinsic to a binding agent. For example, if an antibody is used as binding agent, the Fc portion of the antibody may serve as capturing moiety that can be reversibly captured on protein A (e.g., a protein A-conjugated chromatography column or bead as routinely used in affinity purification) serving as a capturing matrix; or the Fc portion may be captured by another binding agent which specifically binds to the Fc portion (e.g. an anti-Fc antibody). Moreover, if a binding protein serves as binding agent, said binding protein may be expressed with an affinity tag that can be captured using a suitable capturing agent or matrix.

Alternatively, the "capturing moiety" may be present as conjugated on a binding agent. For example, a binding agent (e.g. an antibody) may have conjugated biotin which can be captured by using a biotin-binding "capturing agent or matrix" such as streptavidin- or avidin-coated surface or beads.

The "capturing moiety", according to the present invention, may comprise a "fluorescent label" and/or a "magnetic label" which can be used to conduct the enriching based on a detected fluorescence (e.g. by fluorescence-activated cell sorting (FACS)) or by applying a magnet field (e.g. in magnet-activated cell sorting (MACS)).

The term "fluorescent label" or "fluorescent moiety", as used herein, refers to a compound, chemical group, or composition that is inherently fluorescent. Fluorophores may contain substituents that alter the solubility, spectral properties or physical properties of the fluorophore. Numerous fluorophores are known to those skilled in the art and include, but are not limited, to coumarin, cyanine, benzofuran, a quinoline, a quinazolinone, an indole, a furan, a benzazole, a borapolyazaindacene and xanthenes including fluorescein, rhodamine and rhodol.

The term "magnetic label" or "magnetic moiety", as used herein, refers to a compound, chemical group, or composition that is inherently magnetic.

Suitable fluorescently labeled and/or magnetically labeled binding agents, in particular antibodies (and in particular those having binding specificities useful for the present invention) are either commercially available or can be obtained by known techniques.

The feature that "uncaptured cells are removed" is to be understood that said uncaptured cells are substantially removed, although it is clear to the skilled person, that an absolute (i.e., 100%) removal might not be possible.

In a preferred embodiment of the first aspect of the invention, the one or more T cell-specific activation markers and/or the pro-inflammatory cytokines are solubly expressed (i.e., not membrane-anchored, membrane-bound or membrane associated) and secreted by the antigen-activated T cell. It is understood that for capturing an activated T cell expressing such a secreted activation marker or cytokine, a binding agent may be used which binds to both, the secreted activation marker or cytokine and a membrane-bound (or membrane-anchored) T cell specific cell surface molecule. However, in case the activation marker and/or pro-inflammatory cytokine is itself membrane-bound (or membrane-anchored), i.e., not released into the surrounding medium, a binding agent may be employed which binds to said membrane-bound activation marker and/or pro-inflammatory cytokine and which does not additionally bind to a membrane-bound (or membrane-anchored) T cell specific cell surface molecule.

In a more preferred embodiment of the first aspect of the invention, the T cell-specific pro-inflammatory cytokine is interferon gamma (IFNγ).

In accordance with a preferred embodiment of the first aspect of the invention, the first binding agent is a bi-specific antibody which binds to CD45 and interferon gamma (IFNγ); and/or the second binding agent is an anti-IFNy antibody.

In accordance with an even more preferred embodiment, the first binding agent is a bi-specific antibody which binds to CD45 and interferon gamma (IFNγ); and the second binding agent is an anti-IFNy antibody; and the enriching further comprises applying a third binding agent, wherein the third binding agent is an antibody which (i) binds to the second antibody; and (ii) is conjugated to magnetic beads suitable for magnet-activated cell sorting (MACS).

In accordance with another preferred embodiment of the first aspect of the invention, the PBMCs originate from a donor with detectable virus-specific T-cell frequencies against at least one of said one or more virus-specific antigens; and wherein the donor preferably is at least partially human leukocyte antigen (HLA) matched with a prospective recipient of the T cells.

In those cases where the one or more virus-specific antigens are from multiple (more than one) viruses, the PBMCs preferably originate from a donor with detectable virus-specific T-cell frequencies against at least one of virus-specific antigens of each of said viruses.

The term "virus-specific T-cell frequencies", as used herein, means that there is at least a detectable (meaning here, at least above the detection threshold (i.e. above the limit of detection) population of T cells expressing one or more TCRs specific for one or more virus-specific antigens of said one or more viruses. Methods for assessing a donor's blood sample for the presence or absence of virus-specific T-cells are described in the art for example in Feucht, et al., Blood, (2015) 125 (12):1986-. Icheva et al., J. Clin. Oncol. (2013) Jan 1;31(1):39-48; Feuchtinger et al., Blood (2010) Nov 18;116(20):4360-7.

The term "human leukocyte antigen matching" refers to a process in which blood or tissue samples are tested for human leukocyte antigens (HLAs). HLAs are molecules found on the surface of most cells in the body. They make up a person's tissue type, which varies from person to person. They play an important part in the body's immune response to foreign substances. Human leukocyte antigen matching is done before a donor cell or organ transplant to find out if tissues match between the donor and the person receiving the transplant (i.e., the recipient).

The term "partially human leukocyte antigen (HLA) matched with a prospective recipient", as referred to herein, means that a donor cell (e.g., a donor T cell) expresses at least one HLA allele identical to the HLA alleles of the prospective recipient and at least one HLA allele not identical to the HLA alleles of the prospective recipient. The skilled person understands that a higher degree of "HLA matching" will increase the transplantation tolerance in a recipient of the transplant (i.e., the donor T cell) and decrease the likelihood for immune complications.

In accordance with a preferred embodiment of the first aspect of the invention, the PBMCs are, prior to (a), stimulated *in vitro* with one or more virus-specific antigens of one or more viruses; wherein preferably the virus-specific antigen is a virus-specific protein or protein-glycan-conjugate, more preferable a peptide pool comprising 15-mer peptides corresponding to (preferably overlapping) fragments of a virus-specific protein.

In a particular preferred embodiment of the first aspect of the invention, the virus-specific protein is pp65 of human cytomegalovirus (hCMV), preferably a peptide pool comprising 15-mer peptides corresponding to (optionally overlapping) fragments of hCMV pp65.

In another preferred embodiment, the virus-specific antigen may be selected from one or more AdV-specific proteins (e.g. hexon); EBV-specific proteins (e.g., EBNA, VCA, LMP, BZLF1 and/or BZLF2), BKV-specific proteins (e.g., VP1, VP2 and/or LT); and/or CoV2-specific proteins (including its spike (S), envelope (E), membrane (M), and nucleocapsid (N) proteins).

It was found by the present inventors, that a re-stimulation of the enriched virus-specific T cells before conducting step (b) is beneficial for maintaining the activated state of the enriched virus-specific T cells; and it is believed, without wishing to be bound by theory, that the incorporation of such a re-stimulation step into the method is also productive for enhancing the viability of the generated T cell product. In particular, in the appended experiments, the inventors found that conducting a re-stimulation of the virus-specific T cells (obtained from enrichment, i.e., in step (a)) was particularly effective when conducted immediately after the enrichment step (step (a)), and prior to conducting the GR knockout (step (b)) - by addition/incubation with anti-CD3 and anti-CD28 re-stimulating agent for a duration of 2-6 days (depending on activation status in terms of proliferation clusters visually inspected by microscope).

Thus, in accordance with a preferred embodiment of the first aspect of the invention, the enriched virus-specific T cells obtained in (a) are, prior to (b), re-stimulated *in vitro* with one or more T-cell activating agents.

Suitable "T cell activating agents" may be one or more agonists of a T-cell activating receptor, preferably one or more CD3 agonists and/or CD28 agonists; more preferable one or more anti-CD3 agonistic antibodies and/or anti-CD28 agonistic antibodies.

In a preferred embodiment, the T cell activating agents are anti-CD3 and anti-CD28 agonistic antibodies; most preferably anti-CD3 and anti-CD28 agonistic antibodies conjugated magnetic beads. In the present case, the re-stimulation was particularly effective when using anti-CD3 and anti-CD28 agonistic antibodies conjugated on magnetic beads. However, it is believed that also other T cell activating agents can be used (for example, pro-inflammatory cytokines; and/or virus-specific antigens).

In a further preferred embodiment, a re-stimulation of the enriched virus-specific T cells is conducted by adding the one or more T-cell activating agents after (i.e., preferably immediately or within, with increasing preference, less than 15 hours, 14 hours, 13 hours, 12 hours, 11 hours, 10 hours, 9 hours, 8 hours, 7 hours, 6 hours, 5 hours, 4 hours, 3 hours, 2 hours, 1 hour, 30 min, or more preferable, within less than 15 min after the enriching in step (a) (i.e., after completion of the enriching); and/or for a time period of between, with increasing preference, 12 hours to 15 days, 1 day to 12 days, 1.5 days to 10 days, 1.75 days to 8 days and most preferably between 2 days and 6 days; and at a sufficient dose effective to activate the cells in the respective time period. However, deviations from the indicated time periods may be productive in individual cases, and the skilled person is able to optimize the re-stimulation period, by assessing the activation status by observing proliferation clusters (indicative of activation) e.g. by microscope, whether a longer or shorter re-stimulation might be productive in an individual case. The skilled person is also able to identify a suitable dose of the one or more T-cell activating agents, and to optimizing it e.g. also by assessing the activation by observing the presence of proliferation.

In another preferred embodiment, the T-cell activating agents are separated from the T cells after the re-stimulation, preferably, by removing the T-cell activating agents by applying a capturing step. In an even more preferred embodiment, the T-cell activating agents are (i) conjugated to magnetic beads, and are separated from the T cells after the re-stimulation by applying a magnetic separation.

A particularly preferred gene (or genome) editing method to be employed in accordance with the method of the invention makes use of the nucleases of the clustered regularly interspaced short palindromic repeats (CRISPR/Cas) system.

The CRISPR/Cas system was adapted from a naturally occurring defense system against foreign DNA (e.g. viruses, plasmid DNA) in prokaryotes. Prokaryotes with a CRISPR/Cas system capture fragments of DNA from invading DNA and integrate them into DNA segments known as CRISPR arrays. The CRISPR arrays allow the bacteria/archaea to acquire immunity against the invading DNA (or homologous ones). The bacteria/archaea produce CRISPR-RNAs (crRNAs) from the CRISPR arrays to target the foreign DNA, which in complex with Cas nucleases (also called CRISPR-Cas nucleases or CRISPR nucleases) (e.g. Cas9 or a similar enzyme) inactivate the invading DNA by nucleolytic cleavage.

The CRISPR-Cas system has been harnessed for genome editing in prokaryotes and eukaryotes. A small piece of RNA with a short "guide" sequence (also called herein "spacer element") that attaches (binds) to a specific target sequence of DNA in a genome is created (the so-called guide RNA (gRNA) or single guide (sgRNA)). The genomic target site of the gRNA can be any -20 nucleotide DNA sequence, provided it meets two conditions: (i) The sequence is unique compared to the rest of the genome, and (ii) the target sequence is present immediately adjacent to a Protospacer Adjacent Motif (PAM). The PAM sequence is essential for target binding, but the exact sequence depends on which CRISPR endonuclease is used. CRISPR endonuclease and their respective PAM sequences are known in the art (see https://www.addgene.org/crispr/guide/#pam-table). Hence, the gRNA also binds to the Cas nuclease (e.g. Cas9 or Cpf1). As in bacteria, the gRNA is used to recognize the DNA sequence, and the Cas nuclease cuts the DNA at the targeted location. Once the DNA is cut, the cell's own DNA repair machinery (e.g. NHEJ) adds or deletes pieces of genetic material. Hence, in the CRISPR/Cas system, the CRISPR nuclease makes a double-stranded break (DSB) in DNA at a site determined by the short (∼20 nucleotide) gRNA which break is then repaired within the cell by NHEJ or HDR. The CRISPR/Cas system can be multiplexed by adding multiple gRNAs. It was demonstrated that, for example, five different simultaneous mutations can be introduced into mouse embryonic stem cells by using five different gRNA molecules and one CRISPR endonuclease.

In accordance with a preferred embodiment of the method of the invention, a nucleic acid molecule encoding (in expressible form) a Cas nuclease is introduced into the cells (i.e. the cells enriched in step (a)), whereas in another preferred embodiment of the method of the invention, the Cas nuclease itself (i.e., in proteinaceous form) is introduced into the T cells.

In accordance with another preferred embodiment of the method of the invention, the Cas nuclease itself (i.e., in proteinaceous form) is introduced into the cells, however, in this case in the form of a ribonucleoprotein complex (RNP) together with a suitable guide RNA. RNPs can be assembled *in vitro* and subsequently delivered into the cell by methods known in the art, for example, electroporation or lipofection. RNPs are capable to cleave the target site with comparable efficacy as nucleic acid-based (e.g. vector-based) Cas nucleases (Kim et al. (2014), Genome Research 24(6):1012-1019).

Means for introducing proteins (or peptides) into living cells are known in the art and comprise but are not limited to microinjection, electroporation, lipofection (using liposomes), nanoparticle-based delivery, and protein transduction. Any one of these methods may be used in connection with the invention. In this regard, the Cas (e.g. Cas9) nuclease to be introduced may either be isolated from their natural environment or recombinantly produced.

A liposome used for lipofection is a small vesicle, composed of the same material as a cell membrane (i.e., normally a lipid bilayer e.g. made of phospholipids), which can be filled with one or more protein(s) (e.g. Torchilin VP. (2006), Adv Drug Deliv Rev., 58(14):1532-55). To deliver a protein into a cell, the lipid bilayer of the liposome can fuse with the lipid bilayer of the cell membrane, thereby delivering the contained protein into the cell. It is preferred that the liposomes used in accordance with invention are composed of cationic lipids. The cationic liposome strategy has been applied successfully to protein delivery (Zelphati et al. (2001). J. Biol. Chem. 276, 35103-35110). As known in the art, the exact composition and/or mixture of cationic lipids used can be altered, depending upon the protein(s) of interest and the cell type used (Feigner et al. (1994). J. Biol. Chem. 269, 2550-2561).

Protein transduction specifies the internalization of proteins into the cell from the external environment (Ford et al. (2001), Gene Therapy, 8:1-4). This method relies on the inherent property of a small number of proteins and peptides (preferably 10 to 16 amino acids long) to being able to penetrate the cell membrane. The transducing property of these molecules can be conferred upon proteins which are expressed as fusions with them and thus offer, for example, an alternative to gene therapy for the delivery of therapeutic proteins into target cells. Commonly used proteins or peptides being able to penetrate the cell membrane are, for example; the antennapedia peptide, the herpes simplex virus VP22 protein, HIV TAT protein transduction domain, peptides derived from neurotransmitters or hormones, or a 9xArg-tag.

Microinjection and electroporation are well known in the art and the skilled person knows how to perform these methods. Microinjection refers to the process of using a glass micropipette to introduce substances at a microscopic or borderline macroscopic level into a single living cell. Electroporation is a significant increase in the electrical conductivity and permeability of the cell plasma membrane caused by an externally applied electrical field. By increasing permeability, protein (or peptides or nucleic acid molecules) can be introduced into the living cell.

The Cas nuclease may be introduced into the cells as an active enzyme or as a proenzyme. In the latter case the Cas nuclease is biochemically changed within the cells (for example by a hydrolysis reaction revealing the active site, or changing the configuration to reveal the active site), so that the proenzyme becomes an active enzyme. Means for introducing a nucleic acid molecule encoding in expressible form the Cas nuclease into cells will be discussed in more detail herein below.

In accordance with a preferred embodiment of the first aspect of the invention, the one or more InDels result from non-homologous end joining (NHEJ) DNA repair of one or more targeted double-strand breaks (DSBs), wherein the one or more DSB are mediated by a CRISPR/Cas9 system that is introduced into the virus-specific T cells, wherein the CRISPR/Cas9 system comprises: (i) a Cas9 protein, or a nucleic acid molecule encoding a Cas9 protein; and (ii) a guide RNA (gRNA), or a nucleic acid molecule encoding a gRNA, wherein the gRNA comprises a CRISPR RNA (crRNA) which specifically targets the GR gene; preferably exon 2 of the GR gene (GR-Ex2).

In accordance with a further preferred embodiment of the first aspect of the invention, the CRISPR/Cas9 system is introduced (i.e., delivered) into the virus-specific T cells by means of electroporation, preferably as RNP.

GR has been reported to be expressed in multiple functional isoforms arising from alternative splicing of its nine exons. Exons 2, 3, 4 and 5 and were found to present in the majority of known (human) isoforms (see Petta I, et al., Microbiol Mol Biol Rev. (2016);80(2):495-522). Thus, targeting any of these for introducing disrupting mutations is considered to be a suitable strategy to universally target GR expression in T cells irrespective of the individually expressed isoform. In the experiments conducted in connection with the present invention, it was found that targeting exon 2 was particularly effective in terms of knockout efficiency. However, it is believed that targeting other GR exons, in particular any one of exons 3, 4 and 5 might also be effective and is also particularly envisaged herein.

In accordance with a preferred embodiment of the first aspect of the invention, the Cas9 protein (i) is *Streptococcus pyogenes* (Sp) Cas9 or a homolog thereof; and/or (ii) specifically recognizes the protospacer adjacent motif (PAM) NGG.

In accordance with another (or even more) preferred embodiment of the first aspect of the invention, the crRNA contains a spacer element comprising or consisting of the nucleotide sequence AACCAAAAGUCUUCGCUGCU (SEQ ID NO: 1) or CUUUAAGUCUGUUUCCCCCG (SEQ ID NO: 2).

In connection with the present invention, the above two crRNAs proved particularly (and about equally) effective in terms of knock-out efficiency, and yielded a characteristic donor-independent InDel pattern.

In accordance with a further preferred embodiment of the first aspect of the invention, the time period between (a) and (b) is between about 1 and 14 days, preferably between about 2 to 8 days. The present inventors found that allowing the enriched virus-specific T-cells to rest/recover for a time period of between 2 to 8 days after the enrichment and before conducting the knockout of the GR gene was particularly beneficial and further supportive for the viability. However, it is believed that a also shorter and longer time periods may also be suitable.

In accordance with a different preferred embodiment of the first aspect of the invention, the virus is a pathogenic virus selected from: (a) herpes virus, preferably Cytomegalovirus (CMV), Epstein-Barr virus (EBV), human betaherpesvirus 6 (HHV-6), herpes simplex virus (HSV), human gammaherpesvirus 8 (HHV-8) or adenovirus (AdV); and/or (b) polyomavirus (PyV), preferably BK virus (BKV), JC virus (JCV), Merkel cell virus (MCV), KI virus (KIV), WU virus (WUV), and Simian virus 40 (SV40); and/or (c) retrovirus, preferably lentivirus, more preferably human immunodeficiency virus (HIV); and/or (d) coronavirus (CoV).

In accordance with a further preferred embodiment of the first aspect of the invention, the glucocorticoid is a GR-modulating agent, preferably a glucocorticoid selected from dexamethasone, betamethasone, clobetasol, clobetasone, cortisone, hydroxycortisone, desoxycorticosterone, fludrocortisone, halometasone, fluocortolone, desoximetasone, diflorasone, fluocinonide, flurandrenolide, halobetasol, amcinonide, halocinonide, triamcinolone, hydrocortisone, aclometasone, fluticasone, mometasone, clocortolone, fluocinolone, desonide, prednisone, prednisolone, methylprednisolone and prednicarbate.

The present invention relates in a second aspect to an isolated virus-specific glucocorticoid-resistant T-cell population characterized by (a) having a functionally disrupted endogenous glucocorticoid receptor (GR), wherein the functional disruption is caused by the presence of one or more nucleotide base insertions and/or deletions ('InDels') in the GR coding gene resulting from non-homologous end joining (NHEJ) DNA repair of Cas9-nuclease-induced DNA double-strand breaks (DSBs); and (b) comprising CD8+ cytotoxic T cells and CD4+ helper T cells.

The present invention relates in a third aspect to a virus-specific glucocorticoid-resistant T-cell population obtained by the method according to the first aspect of the invention.

The present invention relates in a forth aspect to a virus-specific glucocorticoid-resistant T-cell population according to the second or third aspect of the invention for use as a medicament.

The term "viral infection", or "virus infection", as interchangeably used herein, refers to the presence of a virus in (or on) a subject's body, which presence has a negative impact on the subject's health or bears a risk for negatively affecting the subject's health.

It will be appreciated that the medical uses and therapeutic/preventive applications of the invention are useful in the fields of human medicine and veterinary medicine. Thus, the "subject", "patient" or "individual" in accordance with the medical uses or treatments of the invention may be a mammal, preferably human, or other animals. For veterinary purposes, subjects include, for example, farm animals such as cows, sheep, pigs, horses, and goats, and poultry such as chickens, turkeys, ducks, and geese; companion animals such as dogs and cats; exotic and/or zoo animals; laboratory animals including mice, rats, rabbits, guinea pigs, and hamsters.

Thus, in accordance with the embodiments of the invention directed to medical uses and therapeutic/preventive applications, the viral infection can involve any one or more (pathogenic or non-pathogenic) viruses capable of infecting a respective subject.

Relevant pathogenic viruses, particularly envisaged by the present invention, yet without being limiting, include: (a) herpes virus, including Cytomegalovirus (CMV), Epstein-Barr virus (EBV), human betaherpesvirus 6 (HHV-6), herpes simplex virus (HSV), human gammaherpesvirus 8 (HHV-8) or adenovirus (AdV); and/or (b) polyomavirus (PyV), including BK virus (BKV), JC virus (JCV), Merkel cell virus (MCV), KI virus (KIV), WU virus (WUV), and Simian virus 40 (SV40); and/or (c) retrovirus, including lentivirus, human immunodeficiency virus (HIV); and/or (d) coronavirus (CoV).

The present invention relates in a fifth aspect to a virus-specific glucocorticoid-resistant T-cell population according to second or third aspect of the invention for use in treating and/or preventing viral infection, or a disorder associated with the viral infection, in a subject undergoing a glucocorticoid-mediated immunosuppressive therapy; wherein preferably the subject is a recipient of (i) solid organ transplantation (SOT), wherein the glucocorticoid-mediated immunosuppressive therapy is for treating or preventing rejection of the transplanted organ; or (ii) allogeneic hematopoietic stem cell transplantation (HSCT), wherein the glucocorticoid-mediated immunosuppressive therapy is for treating or preventing graft-versus-host disease (GvHD); and/or for inducing protective anti-viral T-cell response and/or restoring T-cell-mediated anti-viral immunity, in a subject receiving ongoing glucocorticoid-mediated immunosuppressive therapy.

The present invention relates in a sixth aspect to a method of treating and/or preventing viral infection, or a disorder associated with the viral infection, in a subject undergoing a glucocorticoid-mediated immunosuppressive therapy; wherein preferably the subject is a recipient of (i) solid organ transplantation (SOT), wherein the glucocorticoid-mediated immunosuppressive therapy is for treating or preventing rejection of the transplanted organ; or (ii) allogeneic hematopoietic stem cell transplantation (HSCT), wherein the glucocorticoid-mediated immunosuppressive therapy is for treating or preventing graft-versus-host disease (GvHD); and/or for inducing protective anti-viral T-cell response and/or restoring T-cell-mediated anti-viral immunity, in a subject receiving ongoing glucocorticoid-mediated immunosuppressive therapy.

It is understood that the definitions and embodiments as described above in the context of the first aspect of the invention also apply, in as far as possible, *mutatis mutandis* to the second, third, fourth, fifth and sixth aspects of the present invention.

The invention is herein described, by way of example only, with reference to the accompanying drawings for purposes of illustrative discussion of the preferred embodiments of the present invention.

Unless otherwise defined, all technical and scientific terms used herein have the same meaning as commonly understood by one of ordinary skill in the art to which this invention belongs. In case of conflict, the patent specification, including definitions, will prevail.

Regarding the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The above considerations apply *mutatis mutandis* to all appended claims.

Modifications may be made to the foregoing without departing from the basic aspects of the technology. Although the technology has been described in substantial detail with reference to one or more specific embodiments, those of ordinary' skill in the art will recognize that changes may be made to the embodiments specifically disclosed in this application, yet these modifications and improvements are within the scope and spirit of the technology.

The technology illustratively described herein suitably may be practiced in the absence of any element(s) not specifically disclosed herein. Thus, for example, in each instance herein any of the terms "comprising", "consisting essentially of", and "consisting of" may be replaced with either of the other two terms. The terms and expressions which have been employed are used as terms of description and not of limitation, and use of such terms and expressions do not exclude any equivalents of the features shown and described or portions thereof, and various modifications are possible within the scope of the technology claimed. The terms "method" and "process" are used interchangeably herein.

The term "a" or "an" can refer to one of or a plurality of the elements it modifies (e.g., "a carrier" can mean one or more carriers) unless it is contextually clear either one of the elements or more than one of the elements is described. The term "about" as used herein refers to a value within 10% of the underlying parameter (i.e., plus or minus 10%), and use of the term "about" at the beginning of a string of values modifies each of the values (i.e., "about 1, 2 and 3" refers to about 1, about 2 and about 3). For example, a weight of "about 100 grams" can include weights between 90 grams and 110 grams. Further, when a listing of values is described herein (e.g., about 50%, 60%, 70%, 80%, 85% or 86%) the listing includes all intermediate and fractional values thereof (e.g., 54%, 85.4%). Thus, it should be understood that although the present technology has been specifically disclosed by representative embodiments and optional features, modification and variation of the concepts herein disclosed may be resorted to by those skilled in the art, and such modifications and variations are considered within the scope of this technology.

The Figures show:

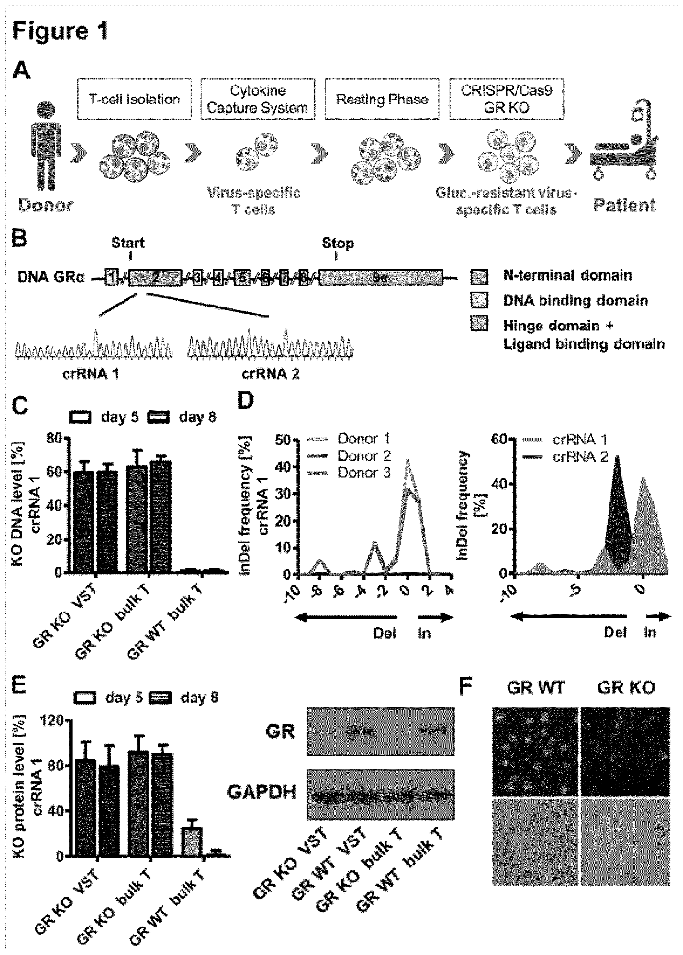

**Figure 1****: CRISPR/Cas9-mediated knockout of glucocorticoid receptor gene**
   **A** Schematic overview of adoptive transfer of virus-specific T cells from a healthy donor to a patient receiving glucocorticoid therapy in order to treat refractory viral infections. Virus-specific T cells were isolated *via* cytokine capture technique ex *vivo* from peripheral blood of a seropositive donor and glucocorticoid receptor (GR) was knocked-out (KO) *via* CRISPR/Cas9. B Location of guide RNAs crRNA1 and crRNA2 binding sites within GRα gene. Chromatograms were obtained *via* Sanger Sequencing. C Confirmation of GR KO efficiency on genetic level. Total frequency of insertions and deletions (InDels) at the CRISPR cut site determined by TIDE webtool on day 5 and day 8 after electroporation. InDels with a p-value < 0.05 in TIDE are depicted. Mean+SD (n=3 donors). D crRNA1 InDel patterns of 3 different donors (left) and InDel patterns of crRNA1 and crRNA2 each of 1 representative donor (right). E GR Knockout efficiency on protein level evaluated *via* Western Blot and ImageJ-based digital image analysis. Protein levels were normalized to those of GAPDH. Mean+SD (n=3 donors). F Immunofluorescence and light microscopy images of GR wildtype (WT) and GR KO T cells stained with GR (G-5) primary and Alexa Fluor 488 secondary antibody.
**Figure 2****: Isolation of virus-specific T cells and phenotypic characterization of the final T-cell product**
   **A-B** Enrichment of IFNγ-secreting T cells from primary human PBMC *via* cytokine capture technique after stimulation with CMV pp65 or SEB (positive control). A Frequency of IFNγ⁺ T cells in the starting material (PBMC) as well as in the negative (bulk T cells) and virus specific T-cell fraction (VST) after magnetic enrichment. Mean+SD (n=3 donors). B Exemplary flow cytometry plots of IFNγ and NLV-multimer staining. **C-E** Characterization of the starting material (PBMC) and the final GR KO virus-specific T-cell product (GR KO VST). **C** Cellular composition and viability analyzed by flow cytometry. Mean (n=4 donors). **D** T-cell phenotype before GR KO (VST), in the final T cell product (GR KO VST) and in wildtype control (GR WT VST) during T-cell production process (day -2 and day 0) and after 3 days (d3) as well as 5 days (d5) of *in vitro* expansion. Mean (n=3 donors). **E** Characterization of flow-cytometric sorted IFNγ⁺ T cells from the final T-cell product. Western Blot and respective ImageJ-based digital image analysis of the KO efficiency (Mean+SD, n=2 donors) and **F** flow-cytometric-based determination of the T-cell phenotype (right, mean, n=3 donors). * p < 0.05, *** p < 0.001, n.s. = not significant.

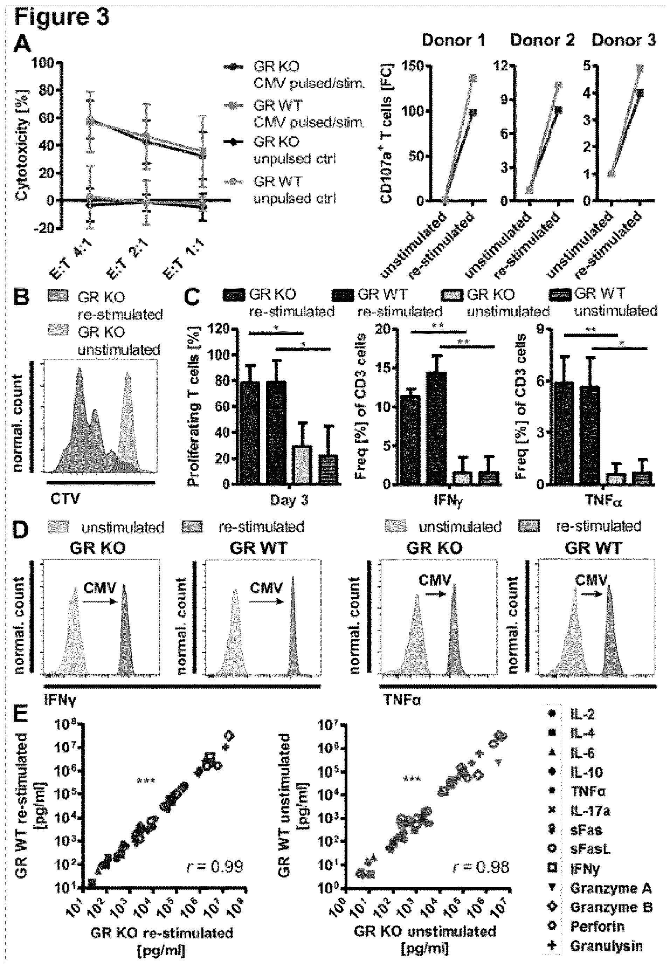

**Figure 3****: Functional characterization of virus-specific GR KO T cells**
   **A** Cytotoxic capacity of CMV-specific GR knockout (GR KO) and CMV-specific GR wildtype (WT) T cells. Cytotoxic capacity against autologous CMV pp65 peptide pulsed PHA blasts upon co-culture in effector to target ratios (E.T) of 4:1; 2:1 and 1:1 (left). Unpulsed PHA blasts served as negative control (unpulsed ctrl). Mean±SD n=3 donors. Fold changes of cytotoxic marker CD107a⁺ T-cell frequencies upon re-stimulation with CMV pp65 peptides in 3 donors (right). **B** Overlay of representative CTV-based proliferation assay histograms on day 3 post CD3/CD28 re-stimulation and unstimulated control. **C** Frequency of proliferating, IFNγ⁺ and TNFα⁺ T cells in CMV pp65 peptide stimulated virus-specific GR KO and GR WT cells as well as the respective unstimulated controls. IFNγ⁺ and TNFα⁺ determined by intracellular cytokine staining. Mean+SD, n=3 donors. **D** Representative IFNγ and TNFα histograms of cell culture supernatant analysis upon CMV pp65 peptide stimulation. **E** Correlation of absolute cytokine concentrations determined *via* multiplex analysis in cell culture supernatants of GR KO and GR WT cells upon CMV pp65 peptide re-stimulation and in the unstimulated setting (n=4 independent experiments of 2 donors). Spearman *r*. * p < 0.05, ** p < 0.01, *** p < 0.0001.
**Figure 4****: Glucocorticoid-resistance of virus-specific GR KO T cells**
   CMV-specific GR knockout (GR KO) and GR wildtype (GR WT) T cells were treated with 200 µM dexamethasone **A** TNFα⁺ T-cell frequencies were determined *via* intracellular cytokine staining in cytotoxic as well as total T cells (n=3 donors). **B** Lymphocyte counts were assessed *via* flowcytometry using absolute quantification mode during 5 days of dexamethasone treatment (n=3 donors) and GR KO rates were determined *via* Western Blot on day 5 of dexamethasone treatment. **C** Exemplary flow-cytometry plots of TNFα intracellular cytokine staining. **D** Absolute cytokine concentrations analyzed in cell culture supernatants. n=2 technical duplicates of 1 donor. **E** Proliferative capacity with and without 400 ng/ml cyclosporine A treatment (n=3 donors). Overlay of representative CTV-based proliferation assay histograms showing CD8⁺ T-cell proliferation on day 3 post re-stimulation. * p < 0.05, ** p < 0.001. Mean ± SEM.

The examples illustrate the invention:

### Example 1: Material and Methods

### Isolation of virus-specific T cells via cytokine capture technique

PBMC were stimulated for 4 hours with Cytomegalovirus (CMV) pp65 Peptivator or Staphylococcus enterotoxin B (SEB) as positive control. IFNγ-secreting cells were isolated *via* IFNγ Secretion Assay kit (Miltenyi Biotec GmbH, Bergisch Gladbach, Germany) according to manufacturer's instructions. In brief, stimulated cells were coated with a CD45-IFNγ bispecific antibody. Bound IFNγ was labelled with an anti-IFNy PE antibody and cells were magnetically enriched *via* an anti-PE antibody conjugated to magnetic beads (Miltenyi Biotec GmbH). PBMC were obtained from healthy individuals after informed consent. Ethics approval was obtained from the local Research Ethics Committee and the study was performed in accordance with the Declarations of Helsinki.

### CRISPR/Cas9-mediated GR knockout

Virus-specific T cells were activated using Dynabeads (anti-CD3/anti-CD28) in a 1:2 bead to cell ratio (Thermo Fisher Scientific Corp., Waltham, USA). Beads were removed using the DynaMAG™-2 magnet and, depending on cell counts, 2 to 8 days after isolation 1x10⁶ cells were electroporated in Buffer 1M (KCI 5 mM, MgCl₂ 15 mM, Na₂HPO₄/NaH₂PO₄ 120 mM, Mannitol 50 mM, pH 7.2)²⁵, mixed with RNP complex containing the respective guideRNA (crRNA 1 AACCAAAAGUCUUCGCUGCU, crRNA 2 CUUUAAGUCUGUUUCCCCCG), Cas9 enzyme and Enhancer according to manufacturer's instructions (all Integrated DNA Technologies, Inc., Skokie, USA). Cells were electroporated in 2 mm cuvettes with pulse code T-023 in an Amaxa Nucleofector IIb (Lonza). After electroporation using Nucleofector II (Amaxa Biosystems GmbH, Cologne, Germany) cells were cultured in TexMACS medium supplemented with 2.5% hAB serum. GR WT cells were cultured and electroporated as GR KO cells, except electroporation without CRISPR/Cas9 reagents (=MOCK control).

### Evaluation of knockout efficiency - TIDE

To evaluate knockout efficiency on genetic level, DNA was isolated using the QIAamp DNA Mini Kit and PCR was performed using forward primer 5'-TCCTGGTAGAGAAGAAAACCCC-3', reverse primer 5'-CCTGCAAAATGTCAAAGGTGC-3' and AccuPrime™ Taq DNA high-fidelity Polymerase (Thermo Fisher Scientific Corp.). PCR products were Sanger sequenced and knockout rate was determined using TIDE as described previously.²⁶

### Western Blot

20 µg protein lysates generated using standard RIPA buffer and supplemented with 6x Laemmli were separated and subsequently blotted onto a nitrocellulose membrane. The membrane was blocked and stained with anti-GR antibody 1:500 - 1:1,000 and anti-GAPDH antibody 1:10,000 (both Santa Cruz Biotechnology Inc., Dallas, USA). Using secondary goat anti-mouse IgG HRP antibody (Santa Cruz Biotechnology Inc.) and Immun-Star HRP Chemiluminescence Kit (BioRad, Hercules, USA) proteins were immunodetected. Blots were quantified using an ImageJ-based digital image analysis approach.

### Immunofluorescence microscopy (IF)

GR knockout cells were fixed with 1% PFA and permeabilized with 0.1% TritonX-100 (both Sigma-Aldrich@, Merck KG, Darmstadt, Germany). Subsequently cells were labelled with GR (G-5) antibody 1:200 (Santa Cruz Biotechnology, Inc.) and stained with secondary goat anti-mouse AlexaFluor® 488 antibody 1:100 (BioLegend, Inc., San Diego, California, USA). Stained cells were applied onto a Poly-L-lysine coated slide (Sigma-Aldrich®, Merck KG, Darmstadt, Germany) under a cover slip with DAPI solution (Thermo Fisher Scientific Corp.).

### Immunosuppressive treatment

Dexamethasone (Sigma-Aldrich®, Merck KG) was added at 200 µM. After 5 days, cells were stained with 7-AAD (BioLegend, Inc.) and quantified using MACSQuant flow cytometer. Cyclosporin A (CsA, Novartis AG, Basel, Switzerland) was added at 400 ng/ml for 5 days. Subsequently cells were labelled with CellTrace Violet Proliferation Kit (Thermo Fisher Scientific Corp.) and re-stimulated with TransAct (Miltenyi Biotec GmbH). Cells were analyzed 24 h, 48 h and 72 h after re-stimulation *via* flow cytometry.

### Intracellular IFNγ staining

Cells were co-cultured with CD3 and CD56 depleted PBMC as feeder cells in a 1:1 ratio and stimulated with CMV pp65 Peptivator (Miltenyi Biotec GmbH). After 2 hours of incubation 10 µg/ml Brefeldin A (Sigma-Aldrich®, Merck KG) was added. 6 hours after re-stimulation cells were stained for viability and CD3-APC-Vio770, CD4-Vio-Bright FITC, CD8-VioBlue, CD56-PE-Vio615, CD45RO-PE-Vio770 and CD62L-APC (Miltenyi Biotec GmbH). Subsequently cells were stained intracellularly with IFNγ-PE (Miltenyi Biotec GmbH) using FIX & PERM™ Cell Permeabilization Kit (Thermo Fisher Scientific Corp.) according to manufacturer's instructions and were measured *via* flow cytometry.

### TNFα, CD107a and CD154 staining

*In vitro* expanded cells were cultured without any cytokines for one day before addition of Brefeldin A and CD107a-APC antibody (Miltenyi Biotec GmbH). After 6 hours of incubation cells were extracellularly stained with CD4-VioGreen, CD3-FITC and CD8-APC/Vio770 antibodies. Subsequently the respective samples were fixated, permeabilized and intracellularly stained using Inside Stain Kit and TNFα-PE-Vio770 and CD154-VioBlue antibodies (all reagents were obtained by Miltenyi Biotec GmbH). Cells were analyzed *via* flow cytometry.

### T-cell phenotype staining

Cells were stained using CD62L-VioBlue, CD45RO-PE-Vio770 and CD95-APC antibodies (all Miltenyi Biotec GmbH) at different time points and analyzed flow cytometrically to distinguish naive T cells (CD45RO⁻ CD62L⁺ CD95⁻), stem-cell like memory T cells (CD45RO⁻ CD62L⁺ CD95⁺), central memory T cells (CD45RO⁺ CD62L⁺ CD95⁺), effector memory T cells (CD45RO⁺ CD62L⁻ CD95⁺) and effector T cells (CD45RO⁻ CD62L⁻ CD95⁺).

### Streptamer staining

Cells were stained with Streptactin PE backbone-labelled Streptamer, either CMV p65 NLVPMVATV or NY-ESO-1 SSLMWITQV as a control (both IBA GmbH, Göttingen, Germany) according to manufacturer's instructions. Cells were washed and additionally stained for CD3-APC-Vio770, CD4-VioBright FITC, CD8-VioBlue, CD56-PE-Vio615 (Miltenyi Biotec GmbH) and viability and analyzed *via* flow cytometry.

### Cytokine release in supernatant

Supernatants were harvested and secreted cytokines were analyzed in a bead-based immunoassay (LegendPlex, Biolegend) according to manufacturer's instructions.

### Proliferation

Cells were labelled with 1 µM CTV for 5 min at 37°C using the CellTrace Violet Proliferation Kit (Thermo Fisher Scientific Corp.) according to manufacturer's instructions. After removal of unbound dye and washing according to manufacturer's instructions 2x10⁵ cells/well were seeded in a 96 well round bottom plate and re-stimulated with 10 µl/ml CD3/CD28 stimulant TransAct. 24 h, 48 h and 72 h after re-stimulation CTV intensity was measured *via* flow cytometry. For evaluation of T-cell proliferation, cells with reduced CTV intensity compared to the 24h measurement were determined as proliferating cells.

### Cytotoxicity Assay

Autologous PBMC were incubated in RPMI medium supplemented with 1% L-Glutamine, 10% FCS and 1 µg/ml PHA (phytohaemagglutinin) for 3 days to induce blast transformation. Subsequently IL-2 was added for 3-5 days. Blasts were then pulsed with 20 µl/ml CMV pp65 Peptivator and labelled with CTV (Miltenyi Biotec GmbH). Virus-specific GR knockout cells were enriched using EasySep™ Human T Cell Enrichment Kit (STEMCELL™ Technologies Inc., Vancouver, Canada) according to manufacturer's instructions. Blasts and effector T cells were co-cultured in TexMACS GMP medium (Miltenyi Biotec GmbH) at different effector-to-target (E:T) ratios for 24 h. Propidium iodide was added 1:100 directly before flow cytometric measurement. For calculation of cytotoxic capacity living target cell count in co-cultured wells was divided by the mean of living target cell count in control wells ('target cells only').

### Example 2: Results

### CRISPR/Cas9-mediated knockout of glucocorticoid receptor gene

In order to generate virus-specific glucocorticoid-resistant T cells, virus-specific T cells were enriched *via* cytokine capture technique followed by CRISPR/Cas9-mediated knockout of the gene NR3C1 (NCBI Reference Sequence: NG_009062.1) encoding the glucocorticoid receptor (GR) **(****Figure 1A****).** Two crRNAs were designed targeting exon 2 of the GR gene coding for the receptor's N-terminal domain **(****Figure 1B****).** The knockout efficiency of the GR was evaluated at day 5 and day 8 *post* electroporation on genetic level *via* PCR and TIDE analysis as well as on protein level *via* Western Blot. Knockout was highly efficient (crRNA1: 59% on genetic and 84% on protein level at day 5) and remained stable over time (59% on genetic and 79% on protein level at day 8; **Figure 1C and E**). Furthermore, the knockout efficiency did not differ significantly between the virus-specific T-cell fraction (=VST) and negative fraction of virus-specific T-cell enrichment (= bulk T cells, 59% ± 7 vs. 62% ± 10 on genetic and 84% ± 17 vs. 91% ± 15 on protein level; **Figure 1C and E**). Investigating CRISPR/Cas9-induced mutation patterns of the crRNAs revealed donor-independent characteristic InDel patterns for the respective crRNAs (**Figure 1D**) although the overall knockout efficiency did not differ significantly. Immunofluorescence microscopy confirmed the GR knockout on protein level determined *via* Western Blot (**Figure 1E and F**)**.**

### Enrichment of virus-specific T cells

For generation of virus-specific T cells, primary human PBMC were stimulated with CMV pp65 and IFNγ-secreting cells were enriched *via* cytokine capture technique. Using CMV pp65 Peptivator, a 15-mer peptide-pool covering the immune-dominant protein pp65 of CMV, virus-specific T cells were enriched significantly (*P* = 0.031) and highly efficiently (163-fold) from mean initial frequencies of 0.3% in the starting material (=PBMC) to 49% in the virus-specific T-cell fraction (=VST; **Figure 2A and B**). Additionally, enrichment of single epitope-specific T-cell clones was analyzed. Specificity against the single peptide pp65 NLVPMVATV was detected using streptamer staining, confirming an enrichment from 0.04% in the original fraction (=PBMC) to 1.97% in the virus-specific T-cell fraction (=VST). This enrichment of NLVPMVATV-specific T-cell clones confirmed virus-specificity of the IFNγ-based enrichment using cytokine capture technique (**Figure 2B**).

For the generation of a glucocorticoid-resistant T-cell product, these virus-specific T cells were further processed by knockout of the glucocorticoid receptor *via* CRISPR/Cas9. In order to characterize the final T-cell product, cellular composition as well as viability was determined by flow cytometry in the starting material (PBMC) and the T-cell product (GR KO VST). **Figure 2C** shows that the starting material consisted of 44% T cells (30% CD4⁺ Tₕₑₗₚₑᵣ cells and 14% CD8⁺ cytotoxic T cells) and other leukocyte subsets such as monocytes, B cells and granulocytes. The virus-specific GR knockout product consisted mainly of T cells (87%) and low frequencies of NKT (2%) and B cells (0.9%). Viability of the starting material as well as the T-cell product was high (98% and 95%, respectively).

To investigate the influence of the manufacturing procedure on T-cell maturation phenotype, frequencies of naive T cells, stem-cell like memory T cells, central memory T cells and effector memory T cells and effector T cells were determined, according to the expression of CD62L and CD45RO and CD95. T cells of freshly isolated PBMC consisted of high frequencies of naive T cells (58%) and lower numbers of effector memory (19%), central memory (15%) and effector cells (7%; **Figure 2D**). After re-stimulation and isolation of virus-specific T cells using cytokine capture technique, the enriched T-cell fraction (VST) showed a mature phenotype with significantly higher frequencies of effector memory (52%) and effector T cells (22%) and significantly lower numbers of naive T cells (7.6%). GR knockout did not influence the T-cell phenotype significantly, confirming that the CRISPR engineering did not alter physiological maturation of virus-specific T cells **(****Figure 2D & E**).

In order to confirm the GR absence in the virus-specific T-cell population of the cell product, IFNγ-positive T cells were further sorted using fluorescence-activated cell sorting (FACS). Subsequent Western Blot analysis confirmed that the knockout efficiency was 97.4% among virus-specific T cells **(****Figure 2E**).

### Functionality of virus-specific GR knockout T cells

In order to assess functionality of the virus-specific GR knockout T cells, cytotoxicity, degranulation marker expression, proliferation and cytokine release patterns upon re-stimulation were investigated. Upon re-stimulation with CMV, using donor-derived pp65 peptide pulsed PHA blasts, GR knockout virus-specific T cells showed similar effector-to-target ratio dependent cytotoxic effects as GR wildtype virus-specific T cells (59% vs. 57% specific target cell lysis; **Figure 3A**). Strong increase of degranulation marker CD107a expression on cytotoxic T cells upon re-stimulation with CMV-specific peptides confirmed the cytotoxicity results and showed donor-dependency (4 to 98-fold; **Figure 3A**). Furthermore, upon re-stimulation 78% of GR knockout virus-specific T cells and 79% of GR wildtype virus-specific T cells proliferated compared to 29% and 22% in the respective unstimulated control samples (**Figure 3B****, C**). Intracellular cytokine staining revealed highly significant up-regulation of IFNγ as well as TNFα secretion (1.5 to 11% and 0.6 to 6%) upon CMV re-stimulation of GR knockout T cells comparable to the up-regulation in GR wildtype cells (1.6 to 14% and 0.7 to 6% respectively; **Figure 3C**).

In order to compare functionality of GR knockout and GR wildtype cells upon CMV re-stimulation in terms of cytokine release, a whole pattern of T_{H1} as well as T_{H2}-cytokines and markers (IL-2, IL-4, IL-6, IL-10, TNFα, IL-17a, sFas, sFasL, IFNγ, granzyme A, granzyme B, perforin and granulysin) were analyzed in the cell culture supernatants *via* multiplex assays. Firstly, strong increase of T_{H1}-cytokines IFNγ and TNFα (**Figure 3D**) upon re-stimulation as well as high absolute concentrations of IFNγ and TNFα in the supernatant of CMV-stimulated T cells confirmed results from intracellular cytokine staining (**Figure 3C and D**)**.** Secondly, highly significant correlation of absolute cytokine/marker levels in GR knockout and GR wildtype cells upon CMV re-stimulation (r = 0.99, p < 0.0001) as well as in the unstimulated situation (r = 0.98, p < 0.0001) confirmed that the GR knockout did not influence functionality of the virus-specific T cells in terms of released cytokine patterns (**Figure 3E**). Highest concentrations were observed for cytoplasmic granule proteases and cytolytic proteins perforin, granzyme A and granzyme B and granulysin (**Figure 3E**).

### Glucocorticoid-resistance of virus-specific GR knockout T cells

In order to investigate glucocorticoid-resistance of GR knockout T cells, cytokine expression, proliferation and levels of GR knockout cells during expansion were analyzed upon high-dose glucocorticoid treatment. GR knockout rescued the inhibitory effect of dexamethasone treatment on virus-specific TNFα-upregulation confirmed by intracellular cytokine staining and absolute quantification of TNFα levels in the cell culture supernatant (**Figure 4A****, C and D**)**.** Intracellular cytokine staining showed that Th1 cytokine expression in T cells like TNFα was up-regulated 9-fold upon dexamethasone treatment in GR KO cytotoxic T cells and 7-fold in GR KO total T cells whereas up-regulation was strongly impaired by dexamethasone treatment in GR wildtype cells (2-fold up-regulation in wildtype cytotoxic and total T cells, **Figure 4A****, C**). Quantification of absolute TNFα levels in the supernatant confirmed the results of intracellular cytokine staining (**Figure 4D**). Additionally, physiological up-regulation of T_{H2}-driving IL-4, pro-inflammatory IL-6 and T-cell activation marker Fas secretion was rescued by GR KO upon dexamethasone treatment (**Figure 4D**)**.** Expansion of lymphocytes in the T-cell product was highly significantly increased by GR knockout during dexamethasone treatment compared to GR wildtype cells (p = 0.002, **Figure 4B**). Western Blot analyses showed that dexamethasone treatment during expansion lead to further enrichment of GR knockout cells **(****Figure 4B****).** As expected, cytotoxicity of GR knockout and GR wildtype T cells was not affected by dexamethasone treatment in our experimental setting (data not shown).

In order to ensure safety in patients with high risk of GvHD, the effect of GR-independent immunosuppressive drugs on GR knockout cells was investigated. Cyclosporine A is a commonly used drug in the *post* transplantation setting exerting its immunosuppressive effect by suppressing T-cell proliferation. Treatment with low dose cyclosporine A lead to suppressed functionality of GR knockout cells comparable with the suppression of wildtype cells, thereby providing a rescue treatment in case of safety issues (**Figure 4E**).

### Discussion

These results demonstrate an efficient CRISPR/Cas9-mediated knockout of the glucocorticoid receptor in virus-specific T cells, leading to glucocorticoid resistance and preserved high antiviral functionality. The processes described here are in line with GMP scale procedures, thereby providing a therapeutic option for the treatment of multiple refractory viral infections after transplantation in patients after SOT or in patients receiving ongoing glucocorticoid therapy due to significant GvHD after HSCT. Enrichment of virus-specific T cells based on cytokine-capture technique, has been already used in clinical practice for decades.⁶ Menger and colleagues provided a proof of concept study for the generation of virus-specific, glucocorticoid resistant T cells by genetic knockout of the glucocorticoid receptor using transcription activator-like effector nuclease (TALEN).²¹ However, Menger et al. performed the genetic knockout in Streptamer-selected virus-specific T cells, an approach which is firstly HLA-restricted, secondly only available for known immunodominant epitopes and thirdly generated T-cell products are restricted to CD8 T cells only. Consequently, this approach has not reached clinical routine application to date. In this study, we combine a clinical available strategy of specific T cell selection (cytokine-capture technique) with fast and feasible CRISPR/Cas9 electroporation. This combinatorial cell engineering overcomes the hurdles of HLA-restriction, limited immunodominant epitopes and is compatible with requirements for GMP grade engineering in clinical studies. Inducing GR-KO T cell responses open a counterintuitive clinical scenario in which immunosuppression could be switched transiently from any other drug to dexamethasone until recovery from the viral infection.

Transient transfection of the CRISPR/Cas9-gRNA ribonucleoprotein (RNP) complex leads to a reduction of off-target effects as well as a reduction of long-term derangement of the cell compared to stable integration of extrinsic protein complexes. Furthermore, the delivery of a fully functional RNP complex *via* electroporation leads to early detectable genetic mutations and more stable efficacies over time as previously described.²² Knockout efficiencies did not differ between virus-specific T cells and bulk T cells. The InDel patterns have been shown to be donor-independent, but highly dependent on the gRNA sequence. Thus, the mutation pattern can be influenced by the design of the respective gRNA.

As previously described ²³ dexamethasone treatment suppresses immune responses, but did not influence cytotoxic capacity of single T cells. Thus, GR KO did not affect cytotoxic capacity of wildtype T cells upon dexamethasone treatment in the present study. In fact, it was previously described that dexamethasone treatment inhibits activation and proliferation rather than T-cell apoptosis.²³ Accordingly, lymphocyte counts did not decrease upon dexamethasone treatment, but CMV-induced increase of lymphocyte counts was inhibited by dexamethasone in wildtype cells. However, lymphocyte counts as well as physiological regulation of TNFα, IL-4, IL-6 and sFas could be rescued by GR KO under high-dose dexamethasone treatment in the present study, confirming the induction of steroid resistance through GR KO in T cells.

Inducing steroid resistance in T cells may carry a risk of uncontrolled T-cell activation. Therefore, a safety concern before clinical application is the sensitivity of the adoptively transferred T cells to other immunosuppressive compounds. Calcineurin-inhibitors like cyclosporine A act independent of the glucocorticoid receptor on the T cells, thereby presenting a potential rescue treatment. The here presented results show that cyclosporine A result in a robust suppression of proliferation in glucocorticoid receptor knockout T cells. General safety issues concerning infusion of CRISPR-KO cells, remain to be investigated in prospective clinical studies. However, recently first in human data have been generated using the same technique in another gene locus treating refractory cancer.²⁴

In conclusion, the generated virus-specific GR knockout T-cell product were found to have high cytolytic activity, specific stimulating properties and high proliferation capacity while remaining resistant to glucocorticoid treatment. Characterization and identification of the product showed a stable effector-memory phenotype and high purities in terms of knockout as well as virus-specificity. Taken together, CRISPR/Cas9-mediated GR engineering of virus-specific T cells is a novel approach for induction of protective T-cell responses against persistent viruses under ongoing steroid-mediated immunosuppression.

### Further References

1. Bahceci, E, Epperson, D, Douek, DC, Melenhorst, JJ, Childs, RC, and Barrett, AJ (2003). Early reconstitution of the T-cell repertoire after non-myeloablative peripheral blood stem cell transplantation is from post-thymic T-cell expansion and is unaffected by graft-versus-host disease or mixed chimaerism. Br J Haematol 122: 934-943.
2. Small, TN, Papadopoulos, EB, Boulad, F, Black, P, Castro-Malaspina, H, Childs, BH, et al. (1999). Comparison of immune reconstitution after unrelated and related T-cell-depleted bone marrow transplantation: effect of patient age and donor leukocyte infusions. Blood 93: 467-480.
3. Federmann, B, Hagele, M, Pfeiffer, M, Wirths, S, Schumm, M, Faul, C, et al. (2011). Immune reconstitution after haploidentical hematopoietic cell transplantation: impact of reduced intensity conditioning and CD3/CD19 depleted grafts. Leukemia 25: 121-129.
4. Hill, JA, Mayer, BT, Xie, H, Leisenring, WM, Huang, ML, Stevens-Ayers, T, et al. (2017). The cumulative burden of double-stranded DNA virus detection after allogeneic HCT is associated with increased mortality. Blood 129: 2316-2325.
5. Riddell, SR, Watanabe, KS, Goodrich, JM, Li, CR, Agha, ME, and Greenberg, PD (1992). Restoration of viral immunity in immunodeficient humans by the adoptive transfer of T cell clones. Science 257: 238-241.
6. Kaeuferle, T, Krauss, R, Blaeschke, F, Willier, S, and Feuchtinger, T (2019). Strategies of adoptive T -cell transfer to treat refractory viral infections post allogeneic stem cell transplantation. J Hematol Oncol 12: 13.
7. Feuchtinger, T, Opherk, K, Bethge, WA, Topp, MS, Schuster, FR, Weissinger, EM, et al. (2010). Adoptive transfer of pp65-specific T cells for the treatment of chemorefractory cytomegalovirus disease or reactivation after haploidentical and matched unrelated stem cell transplantation. Blood 116: 4360-4367.
8. Feucht, J, Opherk, K, Lang, P, Kayser, S, Hartl, L, Bethge, W, et al. (2015). Adoptive T-cell therapy with hexon-specific Th1 cells as a treatment of refractory adenovirus infection after HSCT. Blood 125: 1986-1994.
9. Icheva, V, Kayser, S, Wolff, D, Tuve, S, Kyzirakos, C, Bethge, W, et al. (2013). Adoptive transfer of epstein-barr virus (EBV) nuclear antigen 1-specific t cells as treatment for EBV reactivation and lymphoproliferative disorders after allogeneic stem-cell transplantation. J Clin Oncol 31: 39-48.
10. Matthes-Martin, S, Feuchtinger, T, Shaw, PJ, Engelhard, D, Hirsch, HH, Cordonnier, C, et al. (2012). European guidelines for diagnosis and treatment of adenovirus infection in leukemia and stem cell transplantation: summary of ECIL-4 (2011). Transpl Infect Dis 14: 555-563.
11. Ljungman, P, de la Camara, R, Robin, C, Crocchiolo, R, Einsele, H, Hill, JA, et al. (2019). Guidelines for the management of cytomegalovirus infection in patients with haematological malignancies and after stem cell transplantation from the 2017 European Conference on Infections in Leukaemia (ECIL 7). Lancet Infect Dis 19: e260-e272.
12. Leen, AM, Myers, GD, Sili, U, Huls, MH, Weiss, H, Leung, KS, et al. (2006). Monoculture-derived T lymphocytes specific for multiple viruses expand and produce clinically relevant effects in immunocompromised individuals. Nat Med 12: 1160-1166.
13. Gerdemann, U, Vera, JF, Rooney, CM, and Leen, AM (2011). Generation of multivirus-specific T cells to prevent/treat viral infections after allogeneic hematopoietic stem cell transplant. JVisExp*:* -.
14. Neuenhahn, M, Albrecht, J, Odendahl, M, Schlott, F, Dossinger, G, Schiemann, M, et al. (2017). Transfer of minimally manipulated CMV-specific T cells from stem cell or third-party donors to treat CMV infection after allo-HSCT. Leukemia 31: 2161-2171.
15. Nichols, WG, Corey, L, Gooley, T, Drew, WL, Miner, R, Huang, M, et al. (2001). Rising pp65 antigenemia during preemptive anticytomegalovirus therapy after allogeneic hematopoietic stem cell transplantation: risk factors, correlation with DNA load, and outcomes. Blood 97: 867-874.
16. Cain, DW, and Cidlowski, JA (2017). Immune regulation by glucocorticoids. Nat Rev Immunol 17: 233-247.
17. Levinson, BB, Baxter, JD, Rousseau, GG, and Tomkins, GM (1972). Cellular site of glucocorticoid-receptor complex formation. Science 175: 189-190.
18. Osborn, MJ, Webber, BR, Knipping, F, Lonetree, CL, Tennis, N, DeFeo, AP, et al. (2016). Evaluation of TCR Gene Editing Achieved by TALENs, CRISPR/Cas9, and megaTAL Nucleases. Mol Ther 24: 570-581.
19. Jinek, M, Chylinski, K, Fonfara, I, Hauer, M, Doudna, JA, and Charpentier, E (2012). A programmable dual-RNA-guided DNA endonuclease in adaptive bacterial immunity. Science 337: 816-821.
20. Gasiunas, G, Barrangou, R, Horvath, P, and Siksnys, V (2012). Cas9-crRNA ribonucleoprotein complex mediates specific DNA cleavage for adaptive immunity in bacteria. Proc Natl Acad Sci USA 109: E2579-2586.
21. Menger, L, Gouble, A, Marzolini, MA, Pachnio, A, Bergerhoff, K, Henry, JY, et al. (2015). TALEN-mediated genetic inactivation of the glucocorticoid receptor in cytomegalovirus-specific T cells. Blood 126: 2781-2789.
22. Kosicki, M, Rajan, SS, Lorenzetti, FC, Wandall, HH, Narimatsu, Y, Metzakopian, E, et al. (2017). Dynamics of Indel Profiles Induced by Various CRISPR/Cas9 Delivery Methods. Prog Mol Biol Transl Sci 152: 49-67.
23. Strauss, G, Osen, W, and Debatin, KM (2002). Induction of apoptosis and modulation of activation and effector function in T cells by immunosuppressive drugs. Clin Exp Immunol 128: 255-266.
24. Stadtmauer, EA, Fraietta, JA, Davis, MM, Cohen, AD, Weber, KL, Lancaster, E, et al. (2020). CRISPR-engineered T cells in patients with refractory cancer. Science 367**.**
25. Chicaybam, L, Sodre, AL, Curzio, BA, and Bonamino, MH (2013). An efficient low cost method for gene transfer to T lymphocytes. PLoS One 8: e60298.
26. Brinkman, EK, Chen, T, Amendola, M, and van Steensel, B (2014). Easy quantitative assessment of genome editing by sequence trace decomposition. Nucleic Acids Res 42: e168.

## Claims

1. A method for generating virus-specific glucocorticoid-resistant T cells, comprising:
(a) enriching virus-specific T cells *in vitro* from peripheral blood mononuclear cells (PBMCs), wherein a virus-specific T cell is **characterized by** an elevated expression and/or secretion level of one or more T cell-specific activation markers and/or pro-inflammatory cytokines in response to a prior stimulation with one or more virus-specific antigens as compared to without stimulation with viral-specific antigens; and
(b) disrupting the functionality of the endogenous glucocorticoid receptor (GR) in the enriched virus-specific T cells by introducing one or more nucleotide base insertions and/or deletions ('InDels') into the GR encoding gene.

2. The method according to claim 1, wherein the enriching in (a) comprises:
(i) applying a first binding agent which specifically binds to a T cell-specific surface-molecule and to the one or more activation markers or cytokines;
(ii) applying a second binding agent, which
(ii-1) specifically binds to the activation marker and/or cytokine that is/are bound by the first binding agent; and
(ii-2) comprises a capturing moiety which is capable of reversibly interacting with a capturing agent or matrix; and/or comprises a fluorescent and/or magnetic label for cell sorting; and
(iii) enriching the virus-specific T cells by applying
(iii-1) a capturing method, wherein the virus-specific T cells are captured based on the interaction between the capturing moiety and the capturing agent or matrix; and wherein uncaptured cells are removed; and/or
(iii-2) a cell sorting, wherein the cells are sorted based on the presence of a fluorescent and/or magnetic label.

3. The method according to claim 2, wherein the first binding agent is a bi-specific antibody which binds to CD45 and interferon gamma (IFNγ); and/or the second binding agent is an anti-IFNy antibody.

4. The method according to any one of claims 1 to 3, wherein the PBMCs originate from a donor with detectable virus-specific T-cell frequencies against at least one of said one or more virus-specific antigens; and wherein the donor preferably is at least partially human leukocyte antigen (HLA) matched with a prospective recipient of the T cells.

5. The method according to any one of claims 1 to 4, wherein, prior to (a), the PBMCs are stimulated *in vitro* with one or more virus-specific antigens of one or more viruses; wherein preferably the virus-specific antigen is a virus-specific protein or protein-glycan-conjugate, more preferable a peptide pool comprising 15-mer peptides corresponding to fragments of a virus-specific protein.

6. The method according to any one of claims 1 to 5, wherein the enriched virus-specific T-cells obtained in (a) are, prior to (b), re-stimulated *in vitro* with one or more T-cell activating agents.

7. The method according to any one of claims 1 to 6, wherein the one or more InDels result from non-homologous end joining (NHEJ) DNA repair of one or more targeted double-strand breaks (DSBs), wherein the one or more DSB are mediated by a CRISPR/Cas9 system that is introduced into the virus-specific T cells, wherein the CRISPR/Cas9 system comprises:
(i) a Cas9 protein, or a nucleic acid molecule encoding a Cas9 protein; and
(ii) a guide RNA (gRNA), or a nucleic acid molecule encoding a gRNA, wherein the gRNA comprises a CRISPR RNA (crRNA) which specifically targets the GR gene; preferably exon 2 of the GR gene (GR-Ex2).

8. The method according to any one of claims 1 to 7, wherein the time period between (a) and (b) is between 1 and 14 days, preferably between 2 to 8 days.

9. The method according to any one of claims 1 to 8, wherein the virus is a pathogenic virus selected from:
(a) herpes virus, preferably Cytomegalovirus (CMV), Epstein-Barr virus (EBV), human betaherpesvirus 6 (HHV-6), herpes simplex virus (HSV), human gammaherpesvirus 8 (HHV-8) or adenovirus (AdV); and/or
(b) polyomavirus (PyV), preferably BK virus (BKV), JC virus (JCV), Merkel cell virus (MCV), KI virus (KIV), WU virus (WUV), and Simian virus 40 (SV40); and/or
(c) retrovirus, preferably lentivirus, more preferably human immunodeficiency virus (HIV); and/or
(d) coronavirus (CoV)

10. The method according to any one of claims 1 to 9, wherein the glucocorticoid is a GR-modulating agent, preferably a glucocorticoid selected from dexamethasone, betamethasone, clobetasol, clobetasone, cortisone, hydroxycortisone, desoxycorticosterone, fludrocortisone, halometasone, fluocortolone, desoximetasone, diflorasone, fluocinonide, flurandrenolide, halobetasol, amcinonide, halocinonide, triamcinolone, hydrocortisone, aclometasone, fluticasone, mometasone, clocortolone, fluocinolone, desonide, prednisone, prednisolone, methylprednisolone and prednicarbate.

11. An isolated virus-specific glucocorticoid-resistant T-cell population **characterized by**
(a) having a functionally disrupted endogenous glucocorticoid receptor (GR), wherein the functional disruption is caused by the presence of one or more nucleotide base insertions and/or deletions ('InDels') in the GR coding gene resulting from non-homologous end joining (NHEJ) DNA repair of Cas9-nuclease-induced DNA double-strand breaks (DSBs); and
(b) comprising CD8+ cytotoxic T cells and CD4+ helper T cells.

12. A virus-specific glucocorticoid-resistant T-cell population obtained by the method of any one of claims 1 to 10.

13. A virus-specific glucocorticoid-resistant T-cell population according to claim 11 or 12 for use as a medicament.

14. A virus-specific glucocorticoid-resistant T-cell population according to claim 11 or 12 for use in treating and/or preventing viral infection, or a disorder associated with the viral infection, in a subject undergoing a glucocorticoid-mediated immunosuppressive therapy;
wherein preferably the subject is a recipient of
(i) solid organ transplantation (SOT), wherein the glucocorticoid-mediated immunosuppressive therapy is for treating or preventing rejection of the transplanted organ; or
(ii) allogeneic hematopoietic stem cell transplantation (HSCT), wherein the glucocorticoid-mediated immunosuppressive therapy is for treating or preventing graft-versus-host disease (GvHD);
and/or
for inducing protective anti-viral T-cell response and/or restoring T-cell-mediated anti-viral immunity, in a subject receiving ongoing glucocorticoid-mediated immunosuppressive therapy.


SEQUENCE LISTING

<110> Klinikum der Universität München Anstalt des öffentlichen Rechts

<120> Virus-specific glucocorticoid-resistant T cells for adoptive T-cell
therapy and methods for generating thereof

<160> 2

<170> BiSSAP 1.2

<210> 1
<211> 20
<212> RNA
<213> Artificial Sequence

<220>
<221> source
<222> 1..20
<223> /mol_type="unassigned RNA"
      /note="crRNA 1"
      /organism="Artificial Sequence"

<400> 1
aaccaaaagu cuucgcugcu                                                20


<210> 2
<211> 20
<212> RNA
<213> Artificial Sequence

<220>
<221> source
<222> 1..20
<223> /mol_type="unassigned RNA"
      /note="crRNA 2"
      /organism="Artificial Sequence"

<400> 2
cuuuaagucu guuccccccg                                                20